(19) Europäisches Patentamt
European Patent Office
Office européen des brevets

(11) Publication number : **0 384 350 B1**

(12) **EUROPEAN PATENT SPECIFICATION**

(45) Date of publication of patent specification : **28.04.93 Bulletin 93/17**

(51) Int. Cl.$^5$ : **C07C 65/40,** A61K 31/19, C07C 65/24, C07C 69/94

(21) Application number : **90103143.5**

(22) Date of filing : **19.02.90**

# (54) Dihydroxy naphthalene derivatives.

(30) Priority : **21.02.89 US 313117**
**20.10.89 US 422095**

(43) Date of publication of application :
**29.08.90 Bulletin 90/35**

(45) Publication of the grant of the patent :
**28.04.93 Bulletin 93/17**

(84) Designated Contracting States :
**AT BE CH DE DK FR GB IT LI LU NL SE**

(56) References cited :
**DE-A- 2 745 742**

(73) Proprietor : **F. HOFFMANN-LA ROCHE AG**
**Postfach 3255**
**CH-4002 Basel (CH)**

(72) Inventor : **Carson, Matthew**
**40 Brookfiled Avenue**
**Nutley, N.J. 07110 (US)**
Inventor : **Lee, Han, Ru-Jen**
**17 Colonia Avenue**
**Princeton Junction, N.J. 08550 (US)**
Inventor : **LeMahieu, Ronald Andrew**
**18 Spruce Road**
**North Caldwell, N.J. 07006 (US)**

(74) Representative : **Lederer, Franz, Dr. et al**
**Lederer, Keller & Riederer, Patentanwälte,**
**Lucile-Grahn-Strasse 22**
**W-8000 München 80 (DE)**

EP 0 384 350 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Beschreibung**

Die vorliegende Erfindung betrifft neue Dihydroxynaphthalin-Derivate, ein Vefahren zur Herstellung davon, pharmazeutische Präparate enthaltend diese Derivate, sowie die Verwendung dieser Derivate bei der Herstellung von pharmazeutischen Präparaten.

Die Erfindung betrifft insbesondere Naphthalin-Derivate der Formel

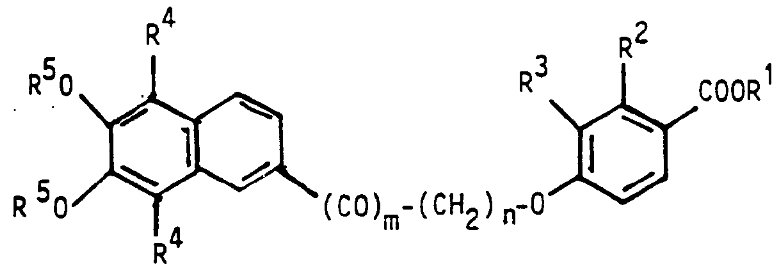

I

worin $R^1$ Wasserstoff, Niederalkyl oder Benzyl, $R^2$ Wasserstoff, Hydroxy oder Niederalkanoyloxy, $R^3$ Wasserstoff oder Niederalkyl, $R^4$ Wasserstoff oder Halogen, $R^5$ Wasserstoff, Acyl, Methyl oder Benzyl, m 0 oder 1, und n eine Zahl zwischen 2 und 10 sind, und Salze davon mit einer pharmazeutisch verwendbaren Base, falls $R^1$ Wasserstoff ist.

Im allgemeinen sind die Verbindungen der Formel I, worin $R^5$ Wasserstoff oder Acyl und $R^1$ Wasserstoff ist, direkt antiinflammatorisch wirksam und als solche verwendbar. Die Verbindungen der Formel I, worin $R^5$ Wasserstoff oder Acyl und $R^1$ Niederalkyl oder Benzyl ist, sind biologische Vorläufer (Prodrugs) von solchen Wirkstoffen, d.h. werden nach der Verabreichung zu den Wirkstoffen hydrolysiert. Die Verbindungen der Formel I, worin $R^5$ Methyl oder Benzyl ist, sind Zwischenprodukte bei der Herstellung der Wirkstoffe der Formel I, in denen $R^5$ Wasserstoff oder Acyl ist.

Im Rahmen der Erfindung bedeutet "Niederalkyl" eine geradkettige oder verzweigte gesättigte Kohlenwasserstoffkette mit 1-7 C-Atome, z.B. Methyl, Aethyl, Propyl, Isopropyl, Butyl, t-Butyl, Neopentyl, Pentyl und Heptyl. Der Ausdruck "Halogen" bezeichnet alle vier Halogene. Der Ausdruck "Acyl" bezeichnet Alkanoylgruppen, die von einer aliphatischen Carbonsäure mit 1-7 C-Atome abgeleitet sind, z.B. Formyl, Acetyl und Propionyl, oder eine Aroylgruppe, die von einer aromatischen Carbonsäure abgeleitet sind, z.B. Benzoyl.

Bevorzugte Verbindungen der Formel I sind diejenigen, worin $R^1$ Wasserstoff, $R^2$ Hydroxy, $R^3$ Niederalkyl, insbesondere Propyl, $R^4$ und $R^5$ Wasserstoff, n 4-6 und m 0 sind.

Besonders bevorzugte Verbindungen der Erfindung sind folgende:

2-Hydroxy-4-[[6-(6,7-dimethoxy-2-naphthalinyl)-6-oxo-hexyl]oxy]-3-propylbenzoesäure;
4-[[6-(6,7-Dihydroxy-2-naphthalinyl)-6-oxo-hexyl]oxy]-2-hydroxy-3-propylbenzoesäure;
2-Hydroxy-4-[6-(6,7-dimethoxy-2-naphthalinyl)hexyloxy]-3-propylbenzoesäure;
4-[6-(6,7-Dihydroxy-2-naphthalinyl)hexyloxy]-2-hydroxy-3-propylbenzoesäure;
2-Hydroxy-4-[4-(6,7-dimethoxy-2-naphthalinyl)butoxy]-3-propylbenzoesäure;
4-[4-(6,7-Dihydroxy-2-naphthalinyl)butoxy]-2-hydroxy-3-propylbenzoesäure;
4-[6-[6,7-Bis(phenylmethoxy)-2-naphthalinyl]hexyloxy]benzoesäure;
4-[6-(6,7-Dihydroxy-2-naphthalinyl)hexyloxy]benzoesäure;
2-Hydroxy-4-[3-(6,7-dimethoxy-2-naphthalinyl)propoxy]-3-propylbenzoesäure;
4-[3-(6,7-Dihydroxy-2-naphthalinyl)propoxy]-2-hydroxy-3-propylbenzoesäure;
2-Hydroxy-4-[2-(6,7-dimethoxy-2-naphthalinyl)äthoxy]-3-propylbenzoesäure;
4-[2-(6,7-Dihydroxy-2-naphthalinyl)äthoxy]-2-hydroxy-3-propylbenzoesäure;
2-Hydroxy-4-[6-[6,7-bis(phenylmethoxy)-2-naphthalinyl]hexyloxy]benzoesäure;
4-[6-(6,7-Dihydroxy-2-naphthalinyl)hexyloxy]-2-hydroxybenzoesäure;
2-Hydroxy-4-[[5-(6,7-dimethoxy-2-naphthalinyl)-5-oxopentyl]oxy]-3-propylbenzoesäure;
4-[[5-(6,7-Dihydroxy-2-naphthalinyl)-5-oxopentyl]oxy]-2-hydroxy-3-propylbenzoesäure;
2-Hydroxy-4-[5-(6,7-dimethoxy-2-naphthalinyl)pentyloxy]-3-propylbenzoesäure;
4-[5-(6,7-Dihydroxy-2-naphthalinyl)pentyloxy]-2-hydroxy-3-propylbenzoesäure;
4-[4-(5,8-Dichloro-6,7-dimethoxy-2-naphthalinyl)butoxy]-2-hydroxy-3-propylbenzoesäure; und
4-[4-(5,8-Dichloro-6,7-dihydroxy-2-naphthalinyl)butoxy]-2-hydroxy-3-propylbenzoesäure;
sowie leicht hydrolysierbare Ester und pharmazeutisch verwendbare Salze von diesen Verbindungen.

Die Verbindungen der Erfindung können dadurch hergestellt werden, dass man ein Halogenid der Formel

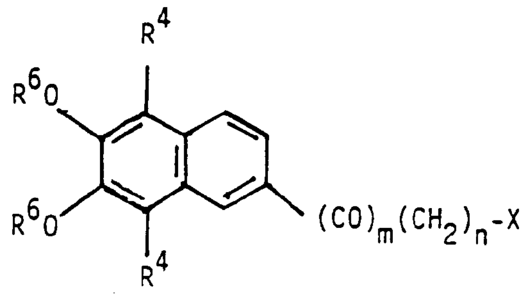

II

worin X Halogen und $R^6$ Methyl, Acyl oder Benzyl ist und $R^4$, m und n die obige Bedeutung haben, mit einer Verbindung der Formel

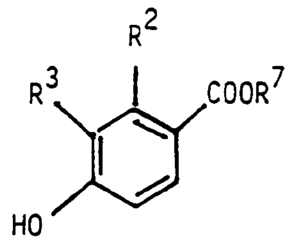

III

worin $R^7$ Niederalkyl oder Benzyl ist und $R^2$ und $R^3$ die obige Bedeutung haben, umsetzt und gewünschtenfalls die Gruppen $-OR^6$, $-OR^7$ und $-(CO)_m(CH_2)_n$ in einer erhaltenen Verbindung der Formel I, worin $R^6$ und $R^7$ für $R^5$ bzw. $R^1$ stehen, funktionell abwandelt.

Die Reaktion der Verbindungen der Formeln II und III wird in Gegenwart einer Base, wie einem Alkalimetallcarbonat, z.B. Natrium- oder Kaliumcarbonat, und von Natrium- oder Kaliumjodid in einem Lösungsmittel, wie Aceton, Methyläthylketon oder Dimethylformamid (DMF) bei einer Temperatur zwischen etwa 40 und 70°C durchgeführt. Tris-[2-(2-methoxyäthoxy)äthyl]amin (TDA-1) kann als Phasentransfer-Katalysator verwendet werden. In diesem Fall wird bei Rückflusstemperatur in einem Lösungsmittel, wie Toluol gearbeitet.

Als fakultative funktionelle Abwandlung einer Gruppe $-OR^7$ kann die Hydrolyse eines Esters der Formel I, worin $R^7$ bzw. $R^1$ Niederalkyl ist, z.B. mit einem Alkalimetall-hydroxid in einem Lösungsmittel, wie Methanol oder Aethanol mit oder ohne Dioxan bei einer Temperatur zwischen etwa 25 und 65°C genannt werden.

Eine Verbindung der Formel I, in der $R^6$ bzw. $R^5$ Methyl ist, kann in die entsprechende Verbindung, worin $R^5$ Wasserstoff ist, übergeführt werden, z.B. durch Behandlung mit Bortribromid in einem Lösungsmittel, wie Methylenchlorid oder 1,2-Dichloräthan, bei einer Temperatur zwischen etwa -70 und +25°C.

In einer Verbindung der Formel I enthaltene Benzylgruppen $R^6$ bzw. $R^5$ können durch Hydrierung bei Raumtemperatur in Gegenwart eines Katalysators, wie Palladium, abgespalten werden. Wenn $R^7$ bzw. $R^1$ auch Benzyl ist, führt die Hydrierung zu einer Säure, worin $R^1$ Wasserstoff ist.

Die Carbonylgruppe in einer Verbindung der Formel I kann zur Methylengruppe hydriert werden, z.B. unter einem Wasserstoffdruck zwischen etwa 3,45 und 4,14 bar, in Gegenwart eines Palladiumkatalysators und eine Mineralsäure, wie Schwefelsäure, in einem Lösungsmittel wie Aethylacetat oder Tetrahydrofuran (THF) bei einer Temperatur zwischen etwa 25 und 70°C.

Die Salze der Säuren der Formel I, worin $R^1$ Wasserstoff ist, können hergestellt werden durch Reaktion dieser Säuren mit einer Base, die ein nicht toxisches pharmakologisch verwendbares Kation enthält. Geeignete Basen sind Alkali- oder Erdalkalimetallhydroxide oder -carbonate, wie Kalzium-, Natrium- oder Kaliumhydroxid oder -carbonat, Ammoniak, primäre, sekundäre oder tertiäre Amine, wie Mono-, Di- oder Trialkylamine, z.B. Methyl-, Diäthyl- oder Trimethylamin, oder stickstoffhaltige cyclische Amine, wie Piperidin.

Die Verbindungen der Formeln II und III können in an sich bekannter Weise hergestellt werden.

So werden Verbindungen der Formel II, worin $R^6$ Methyl ist, durch Acylierung des 6,7-Dimethoxynaphthalins, z.B. mit einem halogenierten Säurechlorid und Aluminiumchlorid in einem Lösungsmittel, wie Methylenchlorid oder 1,2-Dichloräthan, bei einer Temperatur zwischen 0 und 40°C hergestellt. Eine in einer so erhaltenen Verbindung der Formel II, worin $R^4$ Wasserstoff und $R^6$ Methyl ist, enthaltene Carbonylgruppe kann zur Methylengruppe hydriert werden, z.B. wie weiter oben beschrieben für eine Verbindung der Formel I. Die Behandlung der erhaltenen Verbindung, z.B. mit Sulfonylchlorid, in einem halogenierten Kohlenwasserstoff bei einer Temperatur zwischen 0 und 30°C, führt zur entsprechenden Verbindung, worin $R^4$ Chlor ist.

Eine Verbindung der Formel II, worin $R^6$ Benzyl, $R^4$ Wasserstoff und m 0 ist, kann dadurch hergestellt werden, dass man zuerst die entsprechende Verbindung der Formel II, worin $R^6$ Methyl ist, mit Bortribromid in einem halogenierten Kohlenwasserstoff bei einer Temperatur zwischen -75 und 25°C behandelt und die erhaltene Verbindung der Formel II, worin $R^6$ Wasserstoff ist, mit Benzylchlorid oder -bromid, Kalium- oder Natriumjodid und einem Alkalimetallcarbonat, wie Natrium- oder Kaliumcarbonat, in einem Lösungsmittel, wie Aceton oder Methyläthylketon unter Rückfluss, oder mit DMF bei einer Temperatur zwischen 50 und 100°C behandelt.

Eine Verbindung der Formel III, worin $R^2$ Hydroxy, $R^3$ Propyl und $R^7$ Niederalkyl ist, kann dadurch hergestellt werden, dass man die entsprechende Verbindung, worin $R^3$ Wasserstoff ist, allyliert, z.B. unter Verwendung von Allylbromid oder -chlorid, eines Alkalimetallcarbonats wie Natrium- oder Kaliumcarbonat, in einem Lösungsmittel, wie Methyläthylketon, DMF oder Aceton, bei einer Temperatur zwischen 40 und 60°C. Die Umlagerung der erhaltenen Verbindung der Formel III in der eine 2-Propenyloxygruppe an Stelle der Hydroxygruppe steht, zu einer Verbindung der Formel III, in der 2-Propenyl anstelle von $R^3$ steht, wird durch Erhitzen in einer inerten Atmosphäre bei einer Temperatur zwischen 175 und 200°C bewerkstelligt. Die Hydrierung dieser Verbindung der Formel III zu der entsprechenden Verbindung, worin $R^3$ Propyl ist, kann unter Normaldruck oder unter einem Wasserstoffdruck von 3,45 bar in einem Lösungsmittel, wie Aethylacetat, THF oder Aethanol, bei einer Temperatur zwischen 25 und 50°C durchgeführt werden.

Die Verbindungen der Formel I weisen beispielsweise eine Aktivität als $\Delta^5$-Lipoxygenasehemmer auf. Sie sind von Wert als Mittel zur Behandlung von inflammatorischen Krankheiten, wie entzündliche Darmerkrankungen. Das Syndrom der entzündlichen Darmerkrankung umfasst eine Vielzahl von Erkrankungen des Gastrointestinaltrakts, wie Morbus Crohn des Colons und Ileums, ulzerative Colitis und Pseudomembran-Colitis. Gemeinsame Symptome dieser Erkrankungen sind Entzündungen des befallenen Bereiches der Mucosa des Gastrointestinaltraktes, Schleimhautgeschwüre, Oedeme, Infiltration der Mucosa mit Entzündungszellen und schwere Diarrhöe. Arachidonsäuremetaboliten des $\Delta^5$-LO-Weges werden als Mediatoren dieses Syndroms angesehen.

Nachstehend werden verschiedene Testmodelle zur Bestimmung der pharmakodynamischen Aktivität der erfindungsgemässen Verbindungen beschrieben.

IN VITRO TEST FUER $\Delta^5$-LIPOXYGENASEHEMMER

Bei diesem Verfahren wird eine Verbindung auf ihren Effekt auf die $\Delta^5$-Lipoxygenase aus basophilen Leukämiezellen (RBL-1-Zellen) von Ratten geprüft. Die Aktivität des Enzyms wurde durch Messen der katalytischen Umwandlung von [1-$^{14}$C]Arachidonsäure in [1-$^{14}$C]-5-Hydroperoxy-6,8,11,14-Eicosatetraensäure ([1-$^{14}$C]-5-HPETE) bestimmt, die zur Bildung des 5-Hydroxyderivats ([1-$^{14}$C]-5-HETE) führt. Der Test ist in Biochemical Pharmacology 32 (1983) 362 beschrieben.

Bei diesem Test zeigte die 4-[[6-(6,7-Dihydroxy-2-naphthalinyl)-6-oxo-hexyl]oxy] -2-hydroxy-3-propylbenzoesäure ein $IC_{50}$-Wert von 1,2nM und die 4-[4-(6,7-Dihydroxy-2-naphthalinyl)butoxy]-2-hydroxy -3-propylbenzoesäure einen $IC_{50}$-Wert von 4,7nM.

IN VITRO TEST MIT PERITONEALEN MACROPHAGEN VON RATTEN

Dieser Test misst die Fähigkeit der Testverbindung, die Freisetzung von Arachidonsäure (AA) aus Phospholipidspeichern in der Plasmamembran freizusetzen und den anschliessenden Metabolismus der AA auf dem 5-LO und dem Cyclooxygenase (CO)-Weg zu den von den Zellen ausgeschiedenen Endprodukten Leukotrien $B_4$ ($LTB_4$, aus dem 5-LO-Weg) und Prostaglandin $E_2$ ($PGE_2$, aus dem CO-Weg).

Macrophagen wurden von Ratten durch Bauchfellwäsche mit Phosphat-gepufferter Kochsalzlösung minus $Ca^{+2}$ und $Mg^{+2}$ (PBS) erhalten. Die Zellen wurden dreimal mit PBS gewaschen und in Dulbecco's Modified Eagle medium (Gibco Laboratories) suspendiert, das L-Glutamin und D-Glucose enthielt und mit 10% fötalem Kälberserum ergänzt war. Die Zellen wurden mit einem Coulter ZBA-Zähler gezählt und dann in einer Konzentration von 4 x $10^6$ Zellen/ml resuspendiert. 3 ml der Zellsuspension wurden in Plastikkulturschalen (3 cm) gegeben und dann 90 Minuten bei 37°C festsetzen gelassen. Die Schalen wurden dreimal mit PBS gewaschen, um nichthaftende Zellen zu entfernen. $^{14}$C-AA ca. (54µci/Mmol) wurden zugesetzt (1 µCi/Schale). Nach 90 Minuten wurde das nicht aufgenommene $^{14}$C-AA entfernt und die Zellschicht nochmals dreimal mit PBS gewaschen. Die Testverbindungen wurden in DMSO gelöst und mit phosphatgepufferter Hank's Lösung auf die entsprechende Konzentration gebracht. Die Zellen wurden mit der Testverbindung oder mit der zur Lösung der Testverbindungen verwendeten Lösung (Kontrolle) 30 Minuten bei 37°C inkubiert und dann 20 Minuten mit Kalziumionophor A 23187 (5X$10^{-7}$M) stimuliert. Die extracelluläre Flüssigkeit wurde entfernt und die freigesetzte $^{14}$C Radioaktivität aus dem AA Metabolismus durch Flüssigscintillationsspektroskopie gemessen. Die Menge von $LTB_4$ und $PGE_2$ wurden in der extracellulären Flüssigkeit durch Radioimmunoassay mit spezifischen Antiseren gemessen. Die Wirksamkeit einer Testverbindung oder des Standards wurde als %-Hemmung des in Gegenwart von A 23187 produzierten maximalen Effektes berechnet und als 50%-ige Hemmkonzentration ($IC_{50}$) ausgedrückt.

Diese Versuchsanordnung misst die Hemmwirkung der Testverbindungen auf den 5-LO und den CO-Weg des AA-Stoffwechsels und diese Hemmung wird als $IC_{50}$-Wert für $LTB_4$ und $PGE_2$-Bildung ausgedrückt.

In diesem Test zeigte die 4-[[6-(6,7-Dihydroxy-2-naphthalinyl)-6-oxo-hexyl]oxy] -2-hydroxy-3-propylbenzoesäure einen $IC_{50}$-Wert von 3μM für $LTB_4$ und von 2μM für $PGE_2$ und die 4-[4-(6,7-Dihydroxy-2-naphthalinyl)butoxy]-2-hydroxy -3-propylbenzoesäure, einen $IC_{50}$-Wert von 3μM für $LTB_4$ und von 2μM für $PGE_2$-Bildung.

ESSIGSAEURE-INDUZIERTE COLITIS BEI RATTEN IN VIVO

Dieser Test ist in Amer. J. Proc. Gastro. Col. Rec. Surg. 31: 11-18 (1980) und Gastroenterology 88: 55-63 (1985) und 86: 453-460 (1984) beschrieben worden. Die Essigsäure-induzierte Colitis ist gekennzeichnet durch ein Einwandern von Entzündungszellen in das Colon, wobei die Anzahl derartiger Zellen in der Mucosa durch die Aktivität der Myeloperoxidase gemessen wird, eines Marker-Enzyms für diese Zellen. Die wünschenswerte, positive Aktivität wird angezeigt durch eine Verminderung hoher Myeloperoxidasespiegel, die durch Essigsäure bewirkt wird. Männliche Ratten im Gewicht von 150-300 g wurden zweimal täglich während 2 Tagen entweder mit dem Vehikel (Wasser oder Dimethylsulfoxid) oder der geprüften Verbindung, in Wasser suspendiert oder in Dimethylsulfoxid suspendiert, durch orale Verabreichung vor behandelt. Am dritten Tag wurden die Tiere in der gleichen Weise wie an den vorhergehenden beiden Tagen behandelt, mit Metofan anästhesiert, worauf 2 ml 2,5%-ige Essigsäure in das Darmlumen, unmittelbar gefolgt von 3 ml Luft und einer Spülung von 3 ml phosphatgepufferter Kochsalzlösung, injiziert wurden (die Essigsäure ist im Darmlumen hinreichend lange anwesend, um eine Entzündung zu verursachen, ohne jedoch schwere Necrosen oder irreversible Schäden hervorzurufen). Die Tiere erhielten dann eine zweite Dosis der Testverbindung in der gleichen Menge etwa 16 Stunden später. 24 Stunden nach der Behandlung mit Essigsäure wurden die Tiere getötet, die Darmschleimhaut wurde entfernt und bei pH 6 mit einem wässrigen Puffer homogenisiert, worauf die Myeloperoxidase im Homogenat mit o-Phenylendiamin als Chromagen nach dem ELISA-Assay (Zoological Soc., London, 1979, Seiten 29-30) gemessen wurde. Kontrolltiere wurden mit Vehikel und Kochsalzlösung anstelle von Essigsäure behandelt.

Die Messwerte für repräsentative erfindungsgemässe Verbindungen sind in Tabelle I angegeben.

TABELLE I

| Verbindung | Essigsäure-Colitis | |
|---|---|---|
| | Dosis mg/kg p.o. | % Hemmung der Myeloperoxidase-ansammlung |
| 4-[6-(6,7-Dihydroxy-2-naphthalinyl)hexyloxy]-2-hydroxy-3-propylbenzoesäure | 1 | 46±12 |
| 4-[5-(6,7-Dihydroxy-2-naphthalinyl)pentyloxy]-2-hydroxy-3-propylbenzoesäure | 10 | 56±11 |

| | | |
|---|---|---|
| 4-[4-(6,7-Dihydroxy-2-naphthalinyl)butoxy]-2-hydroxy-3-propylbenzoesäure | 3 | 82±8 |
| 4-[3-(6,7-Dihydroxy-2-naphthalinyl)propoxy]-2-hydroxy-3-propylbenzoesäure | 1 | 21±4 |
| 4-[2-(6,7-Dihydroxy-2-naphthalinyl)äthoxy]-2-hydroxy-3-propylbenzoesäure | 10 | 60±13 |
| 4-[6-(6,7-Dihydroxy-2-naphthalinyl)hexyloxy] benzoesäure | 10 | 68±13 |
| 4-[6-(6,7-Dihydroxy-2-naphthalinyl)hexyloxy]-2-hydroxybenzoesäure | 1 | 65±9 |
| 4-[[6-(6,7-Dihydroxy-2-naphthalinyl)-6-oxohexyl] oxy]-2-hydroxy-3-propylbenz-oesäure | 1 | 83±19 |
| 4-[[5-(6,7-Dihydroxy-2-naphthalinyl)-5-oxopentyl]oxy]-2-hydroxy-propylbenzoesäure | 30 | 80±18 |
| 4-[[6-(6,7-Dihydroxy-2-naphthalinyl)-6-oxohexyl]oxy-2-hy-droxy-3-propylbenzoesäure | 1 | 43±11 |
| 4-[4-(5,8-Dichloro-6,7-dihy-droxy-2-naphthalinyl)bu-toxy]-2-hydroxy-3-propylben-zoesäure | 10 | 71±7 |

Die Verbindungen der Formel I oder deren Salze oder Präparate, die eine therapeutisch wirksame Menge an Verbindungen der Formel I oder deren Salze enthalten, können nach an sich bekannten Methoden verabreicht werden, beispielsweise kann eine Verbindung der Formel I oder deren Salz entweder einzeln oder mit anderen pharmazeutischen Mittel, oral, parenteral oder rektal, verabreicht werden. Für die orale Verabreichung können Tabletten, Kapseln, beispielsweise im Gemisch mit Talk, Stärke, Milchzucker oder anderen inerten Inhaltsstoffen, d.h. pharmazeutisch anwendbaren Trägern, in Form von wässrigen Lösungen, Suspensionen, Elixieren oder wässrig alkoholischen Lösungen, beispielsweise im Gemisch von Zucker oder anderen Süsstoffen, Geschmacksstoffen, Farbstoffen, Verdickern und anderen konventionellen pharmazeutischen Excipientien oder als Kügelchen oral verabreicht werden. Für die rektale Verabreichung kommen Suppositorien mit inerten Trägern, wie Kakaobutter in Betracht.

Die zu verabreichende Dosis einer Verbindung der Formel I oder eines Salzes davon und die Häufigkeit der Verabreichung hängt von der Potenz und der Wirkungsdauer der jeweiligen Verbindung der Formel I und dessen Salz ab, wie auch von der gewählten Art der Verabreichung, der Schwere der Erkrankung und des Alters des zu behandelnden Subjekts. Orale Dosen einer Verbindung der Formel I oder eines Salzes davon können im Bereich von etwa 25 bis etwa 1000 mg pro Tag vorzugsweise betragen sie etwa 25 bis etwa 250 mg in einer oder in mehreren Dosen.

In den nachfolgenden Beispielen sind alle Temperaturen in Celsiusgraden angegeben. Sofern nicht anders angegeben, wurden Extrakte über wasserfreiem Magnesiumsulfat getrocknet.

## Beispiel 1

a) Ein Gemisch von 102 g Methyl-2,4-dihydroxybenzoat, 54 ml Allylbromid und 126 g Kaliumcarbonat in 300 ml Aceton wird 3 Stunden unter Rückfluss gerührt. Das Reaktionsgemisch wird filtriert und der Feststoff mit Aceton gewaschen. Nach Entfernung des Acetons aus dem Filtrat unter vermindertem Druck wird der Rückstand destilliert. Man erhält 85 g 2-Hydroxy-4-(2-propenyloxy)benzoesäuremethylester, Siedepunkt 106-108°C/40 Pa.

b) 81 g des Produkts von a) werden unter Argon auf 180-185°C erhitzt. Nach 1,5 Stunden wird die Temperatur 1,5 Stunden auf 210°C erhöht. Nach Abkühlen kristallisiert ein Oel, das nach Umkristallisierung aus Aether/Petroläther 37 g 2,4-Dihydroxy-3-(2-propenyl)benzoesäuremethylester, Smp. 65-66°C, ergibt.

c) Eine Lösung von 54 g des Produkts von b) in 900 ml Aethanol und 3 g von 10% Palladium auf Kohlenstoff wird 45 Minuten unter einer Wasserstoffatmosphäre geschüttelt. Der Katalysator wird abfiltriert und das Filtrat wird unter vermindertem Druck eingeengt. Das entstandene Oel wird mit Hexan verrührt, das Produkt wird filtriert und man erhält 51 g 2,4-Dihydroxy-3-propylbenzoesäuremethylester, Smp. 66-68°C.

d) Eine Lösung von 37 g des Produkts von c) in 57 ml Methanol und 415 ml 3N Natriumhydroxid wird 3 Stunden unter Rückfluss gerührt. Das Methanol wird unter vermindertem Druck entfernt und der Rückstand mit Wasser und 6N Salzsäure behandelt. Das Produkt wird mit Aethylacetat extrahiert und das Extrakt wird getrocknet und unter vermindertem Druck eingeengt. Die erhaltene rohe Säure (35 g), 23 ml Benzylchlorid und 17 g Natriumbicarbonat in 250 ml DMF wird 23 Stunden unter Rühren auf 60°C erhitzt. Das Lösungsmittel wird unter vermindertem Druck entfernt, der Rückstand wird mit gesättigter Natriumbicarbonatlösung behandelt und das Produkt mit Aethylacetat extrahiert. Der trockene Extrakt wird unter vermindertem Druck eingeengt und der Rückstand wird durch Hochdruckflüssigchromatographie (HPCL) mittels 15% Aethylacetat-Hexan gereinigt. Man erhält 36 g 2,4-Dihydroxy-3-propylbenzoesäurebenzylester, Smp. 86-88°C.

e) 2,7 g Aluminiumchlorid werden einer Lösung von 5,8 g 6-Bromhexanoylchlorid in 40 ml Methylenchlorid zugesetzt. Der entstandenen im Eisbad abgekühlten Lösung werden 3,3 g 2,3-Dimethoxynaphthilen zugegeben. Nach einer Stunde Rühren wird das Reaktionsgemisch 17 Stunden bei Raumtemperatur belassen. Wasser wird zugegeben, die organische Schicht wird abgetrennt und mit Natriumbicarbonatlösung gewaschen. Der Extrakt wird getrocknet und unter vermindertem Druck eingeengt. Nach Kristallisierung aus Aethylacetat-Hexan erhält man 3,8 g 6-Brom-1-(6,7-dimethoxy-2-naphthalinyl)-1-hexanon, Smp. 81-82°C.

f) Ein Gemisch von 16,3 g des Produkts von e) und 2 g 10% Palladium auf Kohlenstoff in 200 ml Essigsäure und 4 Tropfen konzentrierte Schwefelsäure wird 22 Stunden unter 3,45 bar Wasserstoff geschüttelt. Das Reaktionsgemisch wird filtriert und das Filtrat unter vermindertem Druck eingeengt. Umkristallisierung aus Aether-Hexan ergibt 9,9 g 2-(6-Bromhexyl)-6,7-dimethoxynaphthalin, Smp. 74-76°C.

g) Zu 9,88 g des Produkts von f) in 200 ml Methylenchlorid, abgekühlt auf -70°C unter Argon, werden 71 ml 1M Bortribromid in Methylenchlorid zugegeben. Das Reaktionsgemisch wird 30 Minuten gerührt und dann 22 Stunden bei -20°C gehalten. Wasser wird zugegeben und die organische Schicht wird abgetrennt und unter vermindertem Druck eingeengt. Der Rückstand wird in Aether gelöst und die Lösung wird mit 1N Salzsäure geschüttelt. Die Aetherschicht wird mit Natriumbicarbonatlösung gewaschen, getrocknet

und unter vermindertem Druck eingeengt. Das Gemisch (8,62 g), 9,5 ml Benzylbromid und 11 g Kaliumcarbonat in Aceton wird unter Rückfluss gerührt. Das Reaktionsgemisch wird unter vermindertem Druck eingeengt und der Rückstand wird mit Aethylacetat-Hexan (1:1) behandelt. Nach Filtrierung wird das Produkt durch HPLC mittels 5% Aethylacetat-Hexan gereinigt und man erhält 8,6 g 2-(6-Bromhexyl)-6,7-bis-(benzoxy)naphthalin, Smp. 64-66°C (Aether-Hexan).

h) Wie weiter oben unter e) beschrieben erhält man aus 27,7 g 5-Brompentanoylchlorid, 26,5 g 5-Brom-1-(6,7-dimethoxy-2-naphthalinyl)-1-pentanon, Smp. 93-95°C (Aceton/Hexan)

i) Wie in e) erhält man aus 21,5 ml 4-Chlorbutyrylchlorid 31,5 g 4-Chlor-1-(6,7-dimethoxy-2-naphthalinyl)-1-butanon, Smp. 98-99°C.

Dieses Produkt wird wie unter f) beschrieben zu 19,6 g 2-(4-Chlorbutyl)-6,7-dimethoxynaphthalin, Smp. 67-69°C, hydriert.

j) Wie unter g) erhält man aus 17,0 g 2-(4-Chlorbutyl)-6,7-dimethoxynaphthalin das 2-(4-Chlorbutyl)-6,7-dihydroxynaphthalin und das 2-(4-Chlorbutyl)-6,7-bis(benzyloxy)naphthalin, Smp. 54-59°C.

k) Wie in e) erhält man aus 3,0 ml 3-Chlorpropionylchlorid 4,70 g 3-Chlor-1-(6,7-dimethoxy-2-naphthalinyl)-1-propanon, Smp 136-137°C. Aus letzterem erhält man wie weiter oben in f) beschrieben 2,88 g 2-(3-Chlorpropyl)-6,7-dimethoxynaphthalin, Smp. 49-53°C..

l) Einer im Eisbad abgekühlten Lösung von 3,0 g 2-(4-Chlorbutyl)-6,7-dimethoxynaphthalin in 25 ml Methylenchlorid werden unter Rühren 1,8 ml Sulfurylchlorid in 10 ml Methylenchlorid zugesetzt. Das Reaktionsgemisch wird 30 Minuten bei 3°C und 6 Stunden bei 24°C gerührt und dann mit Natriumbicarbonatlösung gewaschen, getrocknet und unter vermindertem Druck eingeengt. Reinigung durch HPLC mit 2,5% Aethylacetat-Hexan ergibt 3,32 g 1,4-Dichlor-6-(4-chlorbutyl)-2,3-dimethoxynaphthalin.

m) Ein Gemisch von 3,80 g 6-Brom-1-(6,7-dimethoxy-2-naphthalinyl)-1-hexanon, 2,18 g 2,4-Dihydroxy-3-propylbenzoesäuremethylester, 2,9 g Kaliumcarbonat und 0,3 ml TDA-1 in 80 ml Toluol werden 46 Stunden unter Rühren unter Rückfluss erhitzt. Das Reaktionsgemisch wird filtriert und die Filtrate werden unter vermindertem Druck eingeengt. Umkristallisierung aus Aethylacetat-Hexan ergibt 4,2 g 2-Hydroxy-4[[6-(6,7-dimethoxy-2-naphthalinyl)-6-oxohexyl]oxy]-3-propylbenzoesäuremethylester, Smp. 111-112°C.

Beispiel 2

Eine Lösung von 4,2 g des Produkts von Beispiel 1m) in 100 ml Methanol, 50 ml Dioxan und 34 ml 1N Natriumhydroxid wird 8 Stunden unter Rückfluss gerührt. Das Reaktionsgemisch wird unter vermindertem Druck eingeengt, der Rückstand wird angesäuert und das Produkt wird mit Aethylacetat extrahiert. Der Extrakt wird eingeengt und das Produkt wird aus einem Gemisch von Methanol und Wasser umkristallisiert. Man erhält 3,48 g 2-Hydroxy-4-[6-(6,7-dimethoxy-2-naphthalinyl)-6-oxohexyl]oxy-3-propylbenzoesäure, Smp. 144-146°C.

Beispiel 3

Eine Lösung von 2,0 g des Produkts von Beispiel 2 wird mit Bortribromid behandelt wie beschrieben in Beispiel 1g). Man erhält 1,46 g 4-[[6-(6,7-Dihydroxy-2-naphthalinyl)-6-oxohexyl]oxy]-2-hydroxy-3-propylbenzoesäure, Smp. 205-207°C.

Beispiel 4

Ein Gemisch von 7,60 g 6-Brom-1-(6,7-dimethoxy-2-naphthalinyl)-1-hexanon, 5,96 g 2,4-Dihydroxy-3-propylbenzoesäurebenzylester, 5,75 g Kaliumcarbonat und 3,20 g Natriumjodid in 100 ml Aceton und 25 ml DMF wird 26 Stunden unter Rückfluss gerührt. Die Lösungsmittel werden unter vermindertem Druck entfernt und das Produkt wird durch HPLC mit 2% Aethylacetat-Toluol gereinigt. Man erhält 8,14 g 2-Hydroxy-4[[6,7-dimethoxy-2-naphthalinyl)-6-oxohexyl]oxy]-3-propylbenzoesäurebenzylester.

Beispiel 5

Ein Gemisch von 8,1 g des Produkts von Beispiel 4 und 0,6 g 10% Palladium auf Kohlenstoff in 300 ml THF wird 3 Stunden in einer Wasserstoffatmosphäre geschüttelt. Das Reaktionsgemisch wird filtriert und die Filtrate werden unter vermindertem Druck zu einem Feststoff eingeengt. Umkristallisierung aus Aceton-Wasser ergibt 5,0 g 2-Hydroxy-4-[[6-(6,7-dimethoxy-2-naphthalinyl)-6-oxohexyl]oxy]-3-propylbenzoesäure, Smp. 167-168°C.

Beispiel 6

Ein Gemisch von 1,0 g des Produkts von Beispiel 3, 1,8 ml Aethyljodid und 0,20 g Natriumbicarbonat in 15 ml DMF wird 7 Stunden unter Rühren auf 50°C erhitzt. Das Lösungsmittel wird unter vermindertem Druck entfernt und das Produkt mit Aethylacetat extrahiert. Der getrocknete Extrakt wird eingeengt und das Produkt aus Aceton-Hexan umkristallisiert. Man erhält 0,82 g 4-[[6-(6,7-Dihydroxy-2-naphthalinyl)-6-oxohexyl]oxy]-2-hydroxy-3-propyl-benzoesäureäthylester, Smp. 170-172°C.

Beispiel 7

Analog Beispiel 1f) erhält man durch Hydrierung von 1,5 g des Produkts von Beispiel 2 1,06 g 2-Hydroxy-4-[6-(6,7-dimethoxy-2-naphthalinyl)hexyloxy]-3-propylbenzoesäure, Smp. 102-104°C.

Beispiel 8

Durch Behandlung mit Bortribromid wie in Beispiel 1g) erhält man aus 1,05 g des Produkts von Beispiel 7: 0,72 g 4-[6-(6,7-Dihydroxy-2-naphthalinyl)hexyloxy]-2-hydroxy-3-propylbenzoesäure, Smp. 154-158°C.

Beispiel 9

Durch Umsetzung von 2,38 g 2-(6-Bromhexyl)-6,7-bis(benzoxy)naphthalin und 1,35 g 2,4-Dihydroxy-3-propylbenzoesäurebenzylester wie beschrieben in Beispiel 4 erhält man 2,64 g 2-Hydroxy-4-[6-[6,7-bis(benzoxy)-2-naphthalinyl]hexyloxy]-3-propylbenzoesäurebenzylester, Smp. 94-97°C.

Beispiel 10

Durch Behandlung von 2,6 g des Produkts von Beispiel 9 wie beschrieben in Beispiel 5 erhält man 1,17 g 4-[6-(6,7-Dihydroxy-2-naphthalinyl)hexyloxy]-2-hydroxy-3-propylbenzoesäure, Smp. 165-167°C.

Beispiel 11

Analog Beispiel 4 erhält man durch Umsetzung von 3,16 g 2-(4-Chlorbutyl)-6,7-dimethoxynaphthalin mit 2,34 g 2,4-Dihydroxy-3-propylbenzoesäuremethylester, 4,08 g 2-Hydroxy-4-[4-(6,7-dimethoxy-2-naphthalinyl) butoxy]-3-propylbenzoesäuremethylester.

Beispiel 12

Analog Beispiel 2 erhält man aus 4,05 g des Produkts von Beispiel 11 3,34 g 2-Hydroxy-4-[4-(6,7-dimethoxy-2-naphthalinyl)butoxy]-3-propylbenzoesäure, Smp. 147-148°C.

Beispiel 13

Analog Beispiel 1g) erhält man durch Behandlung mit Bortribromid von 3,3 g des Produkts von Beispiel 12 1,85 g 4-[4-(6,7-Dihydroxy-2-naphthalinyl)butoxy]-2-hydroxy-3-propylbenzoesäure, Smp. 173-175°C.

Beispiel 14

Analog Beispiel 4 erhält man durch Umsetzung von 5,0 g 2-(4-Chlorbutyl)-6,7-bis(benzoxy)naphthalin mit 3,3 g 2,4-Dihydroxy-3-propylbenzoesäurebenzylester 6,2 g, 2-Hydroxy-4-[4-[6,7-bis(benzoxy)-2-naphthalinyl] butoxy]-3-propylbenzoesäurebenzylester, Smp. 87-89°C.

Beispiel 15

Analog Beispiel 5 erhält man aus 6,2 g des Produkts von Beispiel 14 2,75 g 4-[4-(6,7-Dihydroxy-2-naphthalinyl)butoxy]-2-hydroxy-3-propylbenzoesäure, Smp. 176-177°C.

Beispiel 16

Analog Beispiel 4 erhält man durch Umsetzung von 3,3 g 2-(6-Bromhexyl)-6,7-bis(benzoxy)naphthalin mit 1,0 g 4-Hydroxybenzoesäuremethylester, 3,3 g 4-[6-[6,7-Bis(benzoxy)-2-naphthalinyl]hexyloxy]benzoesäuremethylester, Smp. 121-125°C.

Beispiel 17

Analog Beispiel 2 erhält man aus 3,3 g des Produkts von Beispiel 16 3,1 g 4-[6-[6,7-Bis(benzoxy)-2-naphthalinyl]hexyloxy]benzoesäure, Smp. 153-155°C.

Beispiel 18

Analog Beispiel 5 erhält man aus 3,0 g des Produkts von Beispiel 17 1,8 g 4-[6-(6,7-Dihydroxy-2-naphthalinyl)hexyloxy]benzoesäure, Smp. 160-162°C.

Beispiel 19

Analog Beispiel 4 erhält man durch Umsetzung von 3,4 g 2-(3-Chlorpropyl)-6,7-dimethoxynaphthalin und 3,7 g 2,4-Dihydroxy-3-propylbenzoesäurebenzylester, 4,97 g 2-Hydroxy-4-[3-(6,7-dimethoxy-2-naphthalinyl)propoxy]-3-propylbenzoesäurebenzylester, Smp. 94-96°C.

Beispiel 20

Analog Beispiel 5 erhält man aus 4,95 g des Produkts von Beispiel 19 4,1 g 2-Hydroxy-4-[3-(6,7-dimethoxy-2-naphthalinyl)propoxy]-3-propylbenzoesäure, Smp. 165-168°C.

Beispiel 21

Analog Beispiel 1g) erhält man durch Behandlung mit Bortribromid von 4,05 g des Produkts von Beispiel 20 2,98 g 4-[3-(6,7-Dihydroxy-2-naphthalinyl)propoxy]-2-hydroxy-3-propylbenzoesäure, Smp. 218-220°C.

Beispiel 22

Zu 5,0 g im Eisband abgekühltes 2-(2-Hydroxyäthyl)-6,7-dimethoxynaphthalin in 75 ml Methylenchlorid, werden 6 ml Triäthylamin gefolgt von 2,2 ml Methansulfonylchlorid zugegeben. Das Reaktionsgemisch wird 1 Stunde bei 3°C gerührt und dann mit einer Lösung von 1N Salzsäure und 5% Natriumbicarbonat gewaschen. Die organische Schicht wird getrocknet und unter vermindertem Druck eingeengt. Man erhält das 2-(2-Methansulfonyloxyäthyl)-6,7-dimethoxynaphthalin. Ein Gemisch von diesem Mesylat, 5,5 g 2,4-Dihydroxy-3-propylbenzoesäurebenzylester, 0,7 ml TDA-1 und 5,4 g Kaliumcarbonat in 180 ml Toluol wird 18 Stunden unter Rückfluss gerührt. Das Reaktionsgemisch wird mit Wasser gewaschen, getrocknet und unter vermindertem Druck eingeengt. Durch Umkristallisierung des erhaltenen Feststoffes aus Methylenchlorid-Methanol-Wasser erhält man 8,6 g 2-Hydroxy-4-[2-(6,7-dimethoxy-2-naphthalinyl)äthoxy]-3-propylbenzoesäurebenzylester, Smp. 112-113°C.

Beispiel 23

Ein Gemisch von 8,6 g des Produkts von Beispiel 22 und 1,0 g 10% Palladium auf Kohlenstoff in 150 ml Aethylacetat und 100 ml THF wird 4 Stunden unter 37'260 Pa Wasserstoff geschüttelt. Durch Behandlung wie in Beispiel 5 und Umkristallisierung aus Aethylacetat-Hexan erhält man 6,4 g 2-Hydroxy-4-[2-(6,7-dimethoxy-2-naphthalinyl)äthoxy]-3-propylbenzoesäure, Smp. 209-210°C.

Beispiel 24

Analog Beispiel 1g) erhält man durch Behandlung mit Bortribromid von 6,4 g des Produktes von Beispiel 23 0,33 g 4-[2-(6,7-Dihydroxy-2-naphthalinyl)äthoxy]-2-hydroxy-3-propylbenzoesäure, Smp. 216-218°C.

Beispiel 25

Analog Beispiel 4 erhält man durch Umsetzung von 3,3 g 2-(6-Bromhexyl)-6,7-bis(benzoxy)naphthalin und 1,1 g 2,4-Dihydroxybenzoesäuremethylester 3,2 g 2-Hydroxy-4-[6-[6,7-bis(benzoxy)-2-naphthalinyl]hexyloxy] benzoesäuremethylester, Smp. 129-131°C.

Beispiel 26

Eine Lösung von 3,2 g des Produkts von Beispiel 25 in 100 ml Methanol und 60 ml Dioxan und 3,6 ml 6N Natriumhydroxid wird 69 Stunden unter Rückfluss gerührt. Eine Aufarbeitung analog Beispiel 2 ergibt 3,1 g 2-Hydroxy-4-[6-[6,7-bis(benzoxy)-2-naphthalinyl]hexyloxy]benzoesäure, Smp. 149-153°C.

Beispiel 27

Analog Beispiel 5 erhält man ausgehend von 3,1 g des Produktes von Beispiel 26 1,5 g 4-[6-(6,7-Dihydroxy-2-naphthalinyl)hexyloxy]-2-hydroxybenzoesäure, Smp. 190-192°C.

Beispiel 28

Analog Beispiel 4 erhält man durch Umsetzung von 15,0 g 5-Brom-1-(6,7-dimethoxy-2-naphthalinyl)-1-pentanon mit 12,3 g 2,4-Dihydroxy-3-propylbenzoesäurebenzylester 17,6 g 2-Hydroxy-4-[[5,(6,7-dimethoxy-2-naphthalinyl)-5-oxopentyl]oxy]-3-propylbenzoesäurebenzylester, Smp. 126-127°C.

Beispiel 29

Eine Lösung von 15,65 g des Produktes von Beispiel 28 und 38 ml 3N Natriumhydroxid in 400 ml Methanol und 125 ml Dioxan wird 8 Stunden unter Rückfluss gerührt. Aufarbeitung analog Beispiel 2 und Umkristallisierung aus Aceton-Hexan ergeben 12,4 g 2-Hydroxy-4-[[5-(6,7-dimethoxy-2-naphthalinyl)-5-oxopentyl]oxy]-3-propylbenzoesäure, Smp. 177-178°C.

Beispiel 30

Analog Beispiel 1g) erhält man durch Behandlung mit Bortribromid von 5,0 g des Produktes von Beispiel 29 2,6 g 4-[[5-(6,7-Dihydroxy-2-naphthalinyl)-5-oxopentyl]oxy]-2-hydroxy-3-propylbenzoesäure, Smp. 213-214°C.

Beispiel 31

Ein Gemisch von 7,3 g des Produktes von Beispiel 29 und 2,0 g 10% Palladium auf Kohlenstoff in 150 ml THF und 10 ml Essigsäure und 2 Tropfen konzentrierte Schwefelsäure wird 6 Stunden unter 3,73 bar Wasserstoff geschüttelt. Aufarbeitung analog Beispiel 1f) und Umkristallisierung aus Methylenchlorid-Hexan liefern 6,2 g 2-Hydroxy-4-[5-(6,7-dimethoxy-2-naphthalinyl)pentyloxy]-3-propylbenzoesäure, Smp. 135-137°C.

Beispiel 32

Analog Beispiel 1g) erhält man durch Behandlung mit Bortribromid von 6,18 g des Produktes von Beispiel 31 3,0 g 4-[5-(6,7-Dihydroxy-2-naphthalinyl)pentyloxy]-2-hydroxy-3-propylbenzoesäure, Smp. 165-167°C.

Beispiel 33

Analog Beispiel 4 erhält man durch Behandlung von 3,29 g 1,4-Dichlor-6-(4-chlorbutyl)-2,3-dimethoxynaphthalin mit 2,70 g 2,4-Dihydroxy-3-propylbenzoesäurebenzylester 4,28 g 4-[4-(5,8-Dichlor-6,7-dimethoxy-2-naphthalinyl)butoxy]-2-hydroxy-3-propylbenzoesäurebenzylester, Smp. 66-68°C.

Beispiel 34

Analog Beispiel 2 erhält man aus 4,06 g des Produktes von Beispiel 33 3,11 g 4-[4-(5,8-Dichlor-6,7-dimethoxy-2-naphthalinyl)butoxy]-2-hydroxy-3-propylbenzoesäure, Smp. 181-182°C.

Beispiel 35

Analog Beispiel 1g) erhält man durch Behandlung mit Bortribromid von 3,17 g des Produktes von Beispiel 34 1,50 g 4-[4-(5,8-Dichlor-6,7-dihydroxy-2-naphthalinyl)butoxy]-2-hydroxy-3-propylbenzoesäure, Smp. 201-203°C.

Tabletten und Kapseln folgender Zusammenstellung werden in an sich bekannter Weise hergestellt:

## Beispiel A

Tablettenformulierung (Feuchtgranulation)

| Inhaltsstoffe | mg/Tablette 100 mg | 500mg | 1000mg |
|---|---|---|---|
| Produkt von Beispiel 13 | 100 | 500 | 1000 |
| Laktose | 132 | -- | -- |
| Vorgelatinisierte Stärke | 16 | 30 | 50 |
| Modifizierte Stärke | 30 | 40 | 50 |
| Magnesiumstearat | 2 | 6 | 8 |
| TOTAL | 280 | 576 | 1108 |

## Beispiel B

Kapselformulierung

| Inhaltsstoffe | mg/Kapsel | | | |
|---|---|---|---|---|
| Produkt von Beispiel 13 | 25 | 50 | 100 | 500 |
| Wasserhaltige Laktose | 143 | 168 | 148 | -- |
| Maisstärke | 20 | 20 | 40 | 70 |
| Talk | 10 | 10 | 10 | 25 |
| Magnesiumstearat | 2 | 2 | 2 | 5 |
| TOTAL | 200 | 250 | 300 | 600 |

## Beispiel C

### Feuchtgranulat

| Inhaltsstoffe | mg/Tablette | |
|---|---|---|
| Produkt von Beispiel 13 | 25 | 50 |
| Polyvinylpyrrolidon | 5 | 10 |
| Wasserfreie Laktose | 133 | 142 |
| Mikrokristalline Cellulose | 25 | 30 |
| Modifizierte Stärke | 10 | 15 |
| Magnesiumstearat | 2 | 3 |
| TOTAL | 200 | 250 |

## Beispiel D

### Weichgelatinekapsel

| Inhaltsstoffe | mg/Kapsel | |
|---|---|---|
| Produkt von Beispiel 13 | 50 | 150 |
| Polyäthylenglycol 400 | 325 | 550 |
| Mittelkettige Monoglyceride | 100 | 150 |
| Polysorbat 80 | 25 | 50 |
| TOTAL | 500 | 900 |

## Beispiel E

### Kügelchen (für enterale Applikation)

| Bestandteil | mg/Kapsel | | |
|---|---|---|---|
| Produkt des Beispiels 13 | 25 | 100 | 250 |
| Mikrokristalline Cellulose | 100 | 200 | 250 |
| Polyvinylpyrrolidon | 10 | 20 | 30 |
| TOTAL | 135 | 320 | 530 |

In Analogie zu den vorigen Beispielen werden folgende Verbindungen hergestellt:

4-[[5-(6,7-Dihydroxy-2-naphthalinyl)-5-oxopentyl]oxy]-2-hydroxy-3-propylbenzoesäure

4-[[7-(6,7-Dihydroxy-2-naphthalinyl)-7-oxoheptyl]oxy]-2-hydroxy-3-propylbenzoesäure

4-[[8-(6,7-Dihydroxy-2-naphthalinyl)-8-oxooctyl]oxy]-2-hydroxy-3-propylbenzoesäure
4-[[9-(6,7-Dihydroxy-2-naphthalinyl-9-oxononyl]oxy]-2-hydroxy-3-propylbenzoesäure
4-[[6-(6,7-dihydroxy-2-naphthalinyl)-6-oxohexyl]oxy]-2-hydroxybenzoesäure
4-[[6-(6,7-Dihydroxy-2-naphthalinyl)-6-oxohexyl]oxy]-3-propylbenzoesäure
4-[[6-(6,7-Dihydroxy-2-naphthalinyl-6-oxohexyl]oxy]benzoesäure
4-[[6-(6,7-Diacetyloxy-2-naphthalinyl-6-oxohexyl]oxy]-2-hydroxy-3-propylbenzoesäure
4-[[6-(5,8-Dichloro-6,7-dihydroxy-2-naphthalinyl)-6-oxohexyl]oxy]-2-hydroxy-3-propylbenzoesäure
4-[7-(6,7-Dihydroxy-2-naphthalinyl)heptyloxy]-2-hydroxy-3-propylbenzoesäure
4-[8-(6,7-Dihydroxy-2-naphthalinyl)octyloxy]-2-hydroxy-3-propylbenzoesäure
4-[9-(6,7-Dihydroxy-2-naphthalinyl)nonyloxy]-2-hydroxy-3-propylbenzoesäure
4-[10-(6,7-Dihydroxy-2-naphthalinyl)decyloxy-2-hydroxy-3-propylbenzoesäure
4-[6-(5,8-Dichloro-6,7-dihydroxy-2-naphthalinyl)hexyloxy]-2-hydroxy-3-propylbenzoesäure
4-[4-(5,8-Dichloro-6,7-dihydroxy-2-naphthalinyl)butoxy]-2-hydroxybenzoesäure
4-[4-(5,8-Dichloro-6,7-dihydroxy-2-naphthalinyl)butoxy]-2-hydroxy-3-propylbenzoesäureäthylester
4-[4-(6,7-Dihydroxy-2-naphthalinyl)butoxy]-2-hydroxybenzoesäure
4-[4-(6,7-Dihydroxy-2-naphthalinyl)butoxy]benzoesäure
4-[4-(6,7-Dihydroxy-2-naphthalinyl)butoxy]-3-propylbenzoesäure
4-[4-(6,7-Diacetyloxy-2-naphthalinyl)butoxy]-2-hydroxy-3-propylbenzoesäure
4-[4-(6,7-Dihydroxy-2-naphthalinyl)butoxy]-2-hydroxy-3-propylbenzoesäureäthylester
4-[6-(6,7-Dihydroxy-2-naphthalinyl)hexyloxy]-2-hydroxy-3-propylbenzoesäureäthylester

## Patentansprüche

1. Dihydroxynaphthalinderivate der Formel

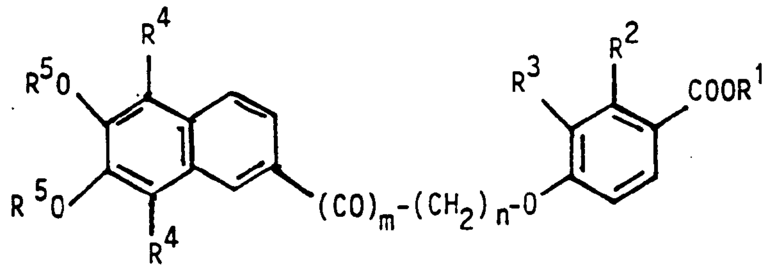

I

worin $R^1$ Wasserstoff, Niederalkyl oder Benzyl, $R^2$ Wasserstoff, Hydroxy oder Niederalkanoyloxy, $R^3$ Wasserstoff oder Niederalkyl, $R^4$ Wasserstoff oder Halogen, $R^5$ Wasserstoff, Acyl, Methyl oder Benzyl, m 0 oder 1, und n eine Zahl zwischen 2 und 10 sind,
und Salze davon mit einer pharmazeutisch verwendbaren Base, falls $R^1$ Wasserstoff ist.

2. Verbindungen nach Anspruch 1, worin $R^1$ Wasserstoff oder Niederalkyl ist.

3. Verbindungen nach Anspruch 1 oder 2, worin $R^1$ Wasserstoff, $R^2$ Hydroxy, $R^3$ Niederalkyl, insbesondere Propyl, $R^4$ und $R^5$ Wasserstoff, n eine Zahl zwischen 4 und 6 und m 0 sind.

4. Verbindungen nach Anspruch 1 aus der Gruppe der folgenden:
4-[6-(6,7-Dihydroxy-2-naphthalinyl)hexyloxy]-2-hydroxy-3-propylbenzoesäure,
4-[5-(6,7-Dihydroxy-2-naphthalinyl)pentyloxy]-2-hydroxy-3-propylbenzoesäure,
4-[4-(6,7-Dihydroxy-2-naphthalinyl)butoxy]-2-hydroxy-3-propylbenzoesäure,
4-[3-(6,7-Dihydroxy-2-naphthalinyl)propoxy]-2-hydroxy-3-propylbenzoesäure,
4-[2-(6,7-Dihydroxy-2-naphthalinyl)äthoxy]-2-hydroxy-3-propylbenzoesäure,
4-[6-(6,7-Dihydroxy-2-naphthalinyl)hexyloxy]benzoesäure,
4-[6-(6,7-Dihydroxy-2-naphthalinyl)hexyloxy]-2-hydroxybenzoesäure,
4-[[6-(6,7-Dihydroxy-2-naphthalinyl)-6-oxohexyl]oxy]-2-hydroxy-3-propylbenzoesäure,
4-[[5-(6,7-Dihydroxy-2-naphthalinyl)-5-oxopentyl]oxy]-2-hydroxy-3-propylbenzoesäure,
4-[[6-(6,7-Dihydroxy-2-naphthalinyl)-6-oxohexyl]oxy]-2-hydroxy-3-propylbenzoesäure,
4-[4-(5,8-Dichloro-6,7-dihydroxy-2-naphthalinyl)butoxy]-2-hydroxy-3-propylbenzoesäure.

5. Die Verbindungen der Ansprüche 1-4 zur Verwendung als Medikamente.

6. Verfahren zur Herstellung der Verbindungen nach einem der Ansprüche 1-4, dadurch gekennzeichnet, dass man ein Halogenid der Formel

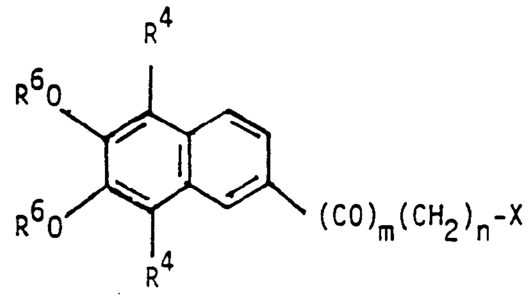

II

worin X Halogen und $R^6$ Methyl, Acyl oder Benzyl ist und $R^4$, m und n die gleiche Bedeutung wie in Anspruch 1 haben,
mit einer Verbindung der Formel

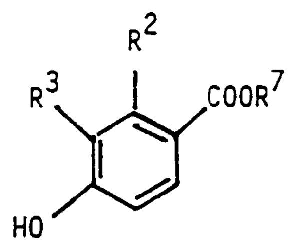

III

worin $R^7$ Niederalkyl oder Benzyl ist und $R^2$ und $R^3$ die gleiche Bedeutung wie in Anspruch 1 haben, umsetzt und gewünschtenfalls die Gruppen $-OR^6$, $-OR^7$ und $-(CO)_m(CH_2)_n-$ in einer erhaltenen Verbindung der Formel I, worin $R^6$ und $R^7$ für $R^5$ bzw. $R^1$ stehen, funktionell abwandelt.

7. Pharmazeutische Präparate enthaltend eine Verbindung nach Anspruch 1 und ein pharmazeutisches Trägermaterial.

8. Die Verwendung einer Verbindung nach Anspruch 1, für die Herstellung eines Medikaments für die Behandlung von inflammatorischen Krankheiten.

## Claims

1. Dihydroxynaphthalene derivatives of the formula

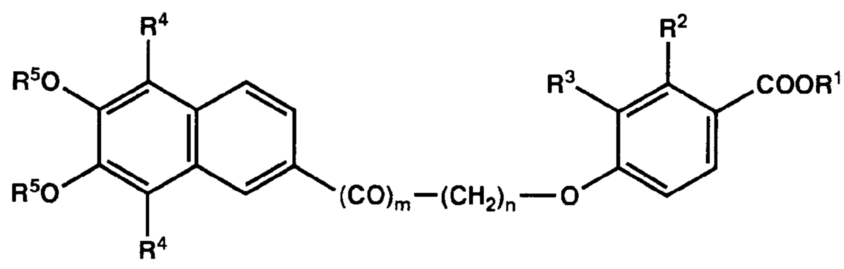

I

in which $R^1$ is hydrogen, lower alkyl or benzyl, $R^2$ is hydrogen, hydroxy or lower alkanoyloxy, $R^3$ is hydrogen or lower alkyl, $R^4$ is hydrogen or halogen, $R^5$ is hydrogen, acyl, methyl or benzyl, m is 0 or 1, and n is an integer between 2 and 10,
and salts thereof with a pharmaceutically usable base when $R^1$ is hydrogen.

2. Compounds according to claim 1, wherein $R^1$ is hydrogen or lower alkyl.

3. Compounds according to claim 1 or 2, wherein $R^1$ is hydrogen, $R^2$ is hydroxy, $R^3$ is lower alkyl, particularly propyl, $R^4$ and $R^5$ are hydrogen, n is an integer between 4 and 6 and m is 0.

4. Compounds according to claim 1 from the following group:
4-[6-(6,7-dihydroxy-2-naphthalenyl) hexyloxy]-2-hydroxy-3-propylbenzoic acid,
4-[5-(6,7-dihydroxy-2-naphthalenyl)pentyloxy]-2-hydroxy-3-propylbenzoic acid,
4-[4-(6,7-dihydroxy-2-naphthalenyl)butoxy]-2-hydroxy-3-propylbenzoic acid,
4-[3-(6,7-dihydroxy-2-naphthalenyl)propoxy]-2-hydroxy-3-propylbenzoic acid,
4-[2-(6,7-dihydroxy-2-naphthalenyl)ethoxy]-2-hydroxy-3-propylbenzoic acid,
4-[6-(6,7-dihydroxy-2-naphthalenyl)hexyloxy]benzoic acid,
4-[6-(6,7-dihydroxy-2-naphthalenyl)hexyloxy]-2-hydroxybenzoic acid,
4-[[6-(6,7-dihydroxy-2-naphthalenyl)-6-oxohexyl]oxy]-2-hydroxy-3-propylbenzoic acid,
4-[[5-(6,7-dihydroxy-2-naphthalenyl)-5-oxopentyl]oxy]-2-hydroxy-3-propylbenzoic acid,
4-[[6-(6,7-dihydroxy-2-naphthalenyl)-6-oxohexyl]oxy]-2-hydroxy-3-propylbenzoic acid,
4-[4-(5,8-dichloro-6,7-dihydroxy-2-naphthalenyl)butoxy]-2-hydroxy-3-propylbenzoic acid.

5. The compounds of claims 1-4 for use as medicaments.

6. A process for the manufacture of the compounds according to any one of claims 1-4, characterized by reacting a halide of the formula

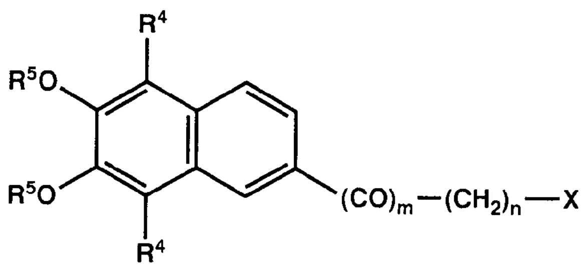

II

wherein X is halogen and $R^6$ is methyl, acyl or benzyl and $R^4$, m and n have the same significance as in claim 1,
with a compound of the formula

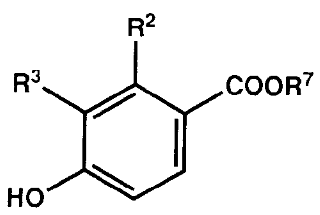

III

wherein $R^7$ is lower alkyl or benzyl and $R^2$ and $R^3$ have the same significance as in claim 1, and, if desired, functionally modifying the groups $-OR^6$, $-OR^7$ and $-(CO)_m(CH_2)_n-$ in a compound of formula I obtained in which $R^6$ and $R^7$ stand for $R^5$ and $R^1$, respectively.

7. Pharmaceutical preparations containing a compound according to claim 1 and a pharmaceutical carrier material.

8. The use of a compound according to claim 1 for the manufacture of a medicament for the treatment of inflammatory diseases.

**Revendications**

1. Dérivés de dihydroxynaphtalène de formule

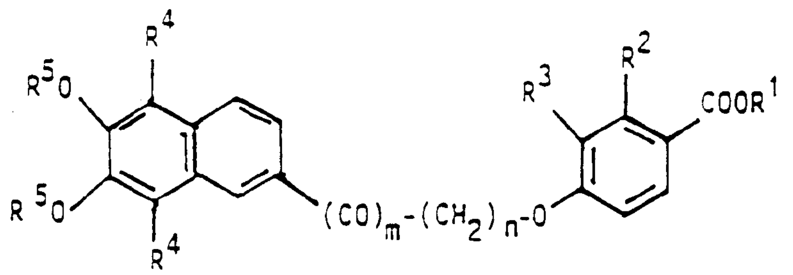

I

où $R^1$ est l'hydrogène, un alkyle inférieur ou le benzyle, $R^2$ est l'hydrogène, l'hydroxy ou un alcanoyle inférieur oxy, $R^3$ est l'hydrogène ou un alkyle inférieur, $R^4$ est l'hydrogène ou un halogène, $R^5$ est l'hydrogène, un acyle, le méthyle ou le benzyle, m est 0 ou 1 et n est un nombre compris entre 2 et 10,
et leurs sels avec une base pharmaceutiquement utilisable, lorsque $R^1$ est l'hydrogène.

2. Composés selon la revendication 1 où $R^1$ est l'hydrogène ou un alkyle inférieur.

3. Composés selon la revendication 1 ou 2 où $R^1$ est l'hydrogène, $R^2$ l'hydroxy, $R^3$ un alkyle inférieur, en particulier le propyle, $R^4$ et $R^5$ sont l'hydrogène, n est un nombre compris entre 4 et 6 et m est 0.

4. Composés selon la revendication 1, provenant du groupe des composés suivants :
   l'acide 4-[6-(6,7-dihydroxy-2-naphtalényl)hexyloxy]-2-hydroxy-3-propylbenzoïque,
   l'acide 4-[5-(6,7-dihydroxy-2-napthtalényl)pentyloxy]-2-hydroxy-3-propylbenzoïque,
   l'acide 4-[4-(6,7-dihydroxy-2-naphtalényl)butoxy]-2-hydroxy-3-propylbenzoïque,
   l'acide 4-[3-(6,7-dihydroxy-2-napthalényl)propoxy]-2-hydroxy-3-propylbenzoïque,
   l'acide 4-[2-(6,7-dihydroxy-2-naphtalényl)éthoxy]-2-hydroxy-3-propylbenzoïque,
   l'acide 4-[6-(6,7-dihydroxy-2-naphtalényl)hexyloxy]-benzoïque,
   l'acide 4-[6-(6,7-dihydroxy-2-naphtalényl)hexyloxy]-2-hydroxy-benzoïque,
   l'acide 4-[[6-(6,7-dihydroxy-2-naphtalényl)-6-oxohexyl]oxy]-2-hydroxy-3-propylbenzoïque,
   l'acide 4-[[5-(6,7-dihydroxy-2-napthalényl)-5-oxopentyl]oxy]-2-hydroxy-3-propylbenzoïque,
   l'acide 4-[[6-(6,7-dihydroxy-2-napthalényl)-6-oxohexyl]oxy]-2-hydroxy-3-propylbenzoïque,
   l'acide 4-[4-(5,8-dichloro-6,7-dihydroxy-2-napthalényl)butoxy]-2-hydroxy-3-propylbenzoïque.

5. Les composés des revendications 1 à 4, destinés à être utilisés comme médicaments.

6. Procédé pour la préparation des composés selon l'une des revendications 1 à 4, caractérisé en ce que l'on fait réagir un halogénure de formule

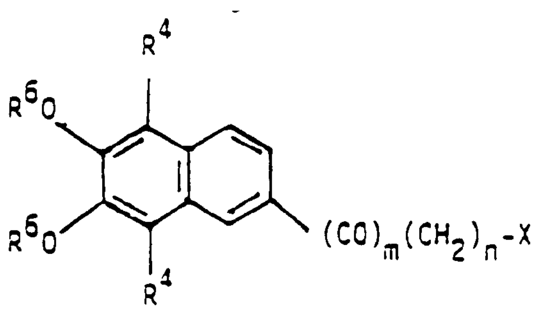

II

où X est un halogénure et $R^6$ est le méthyle, un acyle ou le benzyle et $R^4$, m et n ont la même signification que dans la revendication 1,
avec un composé de formule

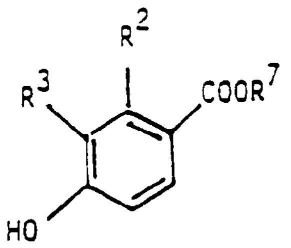

III

où $R^7$ est un alkyle inférieur ou le benzyle et $R^2$ et $R^3$ ont la même signification que dans la revendication 1 et, si désiré, en ce que l'on transforme fonctionnellement les groupes $-OR^6$, $-OR^7$ et $-(CO)_m(CH_2)_n-$ dans le composé obtenu de formule I où $R^6$ et $R^7$ représentent $R^5$ ou $R^1$.

7. Préparations pharmaceutiques contenant un composé selon la revendication 1 et un support pharmaceutique.

8. Utilisation d'un composé selon la revendication 1 pour la préparation d'un médicament destiné au traitement des maladies inflammatoires.